(19) Europäisches Patentamt / European Patent Office / Office européen des brevets

(11) **EP 4 289 369 B1**

(12) **EUROPEAN PATENT SPECIFICATION**

(45) Date of publication and mention
of the grant of the patent:
**27.08.2025 Bulletin 2025/35**

(21) Application number: **23175953.1**

(22) Date of filing: **30.05.2023**

(51) International Patent Classification (IPC):
***A61M 25/00*** (2006.01)  ***A61B 10/00*** (2006.01)
***A61B 10/04*** (2006.01)

(52) Cooperative Patent Classification (CPC):
**A61B 10/0051;** A61B 10/04; A61B 2010/0061;
A61M 25/007

(54) **SAMPLING CATHETER**

PROBENAHMEKATHETER

CATHÉTER D'ÉCHANTILLONNAGE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **07.06.2022 GB 202208350**

(43) Date of publication of application:
**13.12.2023 Bulletin 2023/50**

(73) Proprietors:
• **Hunt Developments (UK) Ltd.
Midhurst
West Sussex GU29 9HX (GB)**
• **Imperial College Innovations Limited
London SW7 2AZ (GB)**

(72) Inventors:
• **HUNT, Toby
West Sussex, GU29 9HX (GB)**
• **HUNT, Duncan
West Sussex, GU29 9HX (GB)**
• **HUNT, Trevor
West Sussex, GU29 9HX (GB)**
• **HANSEL, Trevor Thomas
London, SW7 2AZ (GB)**

(74) Representative: **Lewis Silkin LLP
Arbor
255 Blackfriars Road
London SE1 9AX (GB)**

(56) References cited:
WO-A1-2013/084945    US-A- 3 945 385
US-A- 5 643 230    US-A- 5 788 680
US-A1- 2007 093 727    US-A1- 2021 244 623

## Description

## Introduction

The present invention relates to a sampling catheter for obtaining a sample of mucosal lining fluid from the respiratory tract, especially the lower respiratory tract, or the upper gastrointestinal tract. More particularly, the invention relates to a suction catheter.

## Background to the Invention

Many medical conditions, especially respiratory conditions, can be diagnosed by detecting the presence and quantities of biomarkers, for example:

- cells such as eosinophils and neutrophils and their granule proteins, cytokines (such as IL-4, IL-5, IL-9 and IL-13), prostanoids, and mucosal IgE which are indicative of inflammatory conditions such as asthma and allergies, chronic obstructive pulmonary disease (COPD) and infection;
- markers of infection, including bacterial infection (for example with *Streptococcus, Pseudomonas, Mycobacteria*), viral infection and fungal infection, associated cells, nucleic acids, antibodies, interferons, acute phase reactants (APR), cytokines/chemokines;
- pathogens (especially *Pseudomonas*), mucus characteristics and electrolytes in cystic fibrosis;
- biomarkers of pulmonary fibrosis, airway remodelling, complement activation and coagulation, lung transplant rejection and autoimmune lung disease;
- biomarkers of pulmonary vascular disease, including pulmonary artery hypertension (PAH), cardiac failure and acute respiratory distress syndrome (ARDS);
- metabolic markers such as pH and glucose levels, which can indicate acid reflux, diabetes mellitus and can be altered in infection;
- biomarkers of cancer and precancerous states such as tumour-specific DNA, micro-RNA and other small RNA species, tumour cells;
- measures of levels of drugs and their metabolites, including pharmacokinetics and pharmacodynamics of drug action over time.

The biomarkers above can also be measured in experimental animals such as mice, rats, guinea pigs, ferrets, primates and domestic and farm animals.

However, it has proved particularly difficult to quantitate biomarkers which are indicators of respiratory diseases. Blood, sputum and breath samples have all been used to measure biomarkers of inflammation in patients with respiratory diseases: but all these samples have deficiencies, and this has provided the impetus to study the surface fluid on the airway mucosa[1]. In summary, blood is too far from the site of airway disease, sputum contains many dead and dying cells in a thick mucus, while both sputum and breath are contaminated by saliva. There is therefore a need to obtain airway samples of adequate volume from the lower respiratory tract, including the trachea and bronchi, with a known volume of undiluted sample. It is preferable that the sample should be obtained using a non-invasive method.

Conventional methods of taking a sample from the respiratory tract include nasopharyngeal sampling, in which a sample is taken from the upper respiratory tract, and bronchoscopy, where a sample is obtained from the bronchus using techniques such as bronchial biopsy, bronchoalveolar lavage (BAL), bronchial brushing, and bronchial microsampling.

Recent non-invasive approaches to breath and sputum analysis include the absorption/adsorption of mucosal lining fluid from the airway using Nasosorption™ and Bronchosorption™ sampling devices (available from Hunt Developments (UK) Limited, Midhurst, West Sussex, UK). These devices employ swabs comprising a synthetic absorbent/adsorbent matrix (SAM) and devices and methodology have been developed for clinical sampling, as well as for elution of the absorbed mucosal lining fluid[2].

Oropharyngeal and nasopharyngeal sampling are simple techniques which can be used for all patient groups including babies and young children. Samples can be obtained easily in a non-specialist setting such as a general practitioner's surgery and, in some cases, self-sampling can be carried out by the patient.

The eluate from oropharyngeal and nasopharyngeal sampling, especially from Nasosorption™, can be used to measure a wide variety of parameters: cytokines and chemokines, prostanoids, leukocyte granule proteins, complement, antibodies, nucleic acids and viruses. Nasosorption™ samples typically produce higher detectable levels of cytokines compared to nasal lavage, and many inflammatory biomarkers are at higher levels in mucosal lining fluid than in blood.

For example, in studies of allergy using samples obtained by Nasosorption™, it was possible to show differences in type 2 cytokines (IL-5 and IL-13) in allergic and non-allergic children[3]. In addition, it was shown that cytokines in Nasosorption™ samples from neonates are influenced by maternal atopy[4], and asymptomatic picornavirus infection in neonates also alters Nasosorption™ inflammatory mediators[5]. More recently, in adults with asthma, it has been shown that Nasosorption™ IL-5 is correlated with sputum eosinophils[6].

Clinical studies have also been carried out with grass pollen nasal allergen challenge, followed by repeated Nasosorption™ sampling to characterise the kinetics of the nasal mucosal immune response in terms of molecular biomarkers[7,8,9]. This mimics natural hay fever and it is possible to study the molecular basis of early mast cell activation (up to 1 hour) and late-type 2 (eosinophilic) inflammation (from 2 to 12 hours). Monoclonal antibody against IL-13 (anti-IL-13) has been shown to inhibit nasal mediator levels following nasal allergen challenge[10]. Lipopolysaccharide nasal challenge has been performed in healthy volunteers, showing that this powerful bacterial immunogen can cause an IL-1β and IL-6 response[11].

Nasosorption™ and Bronchosorption™ sampling have also been used in studies of respiratory RNA viral infection. Human rhinovirus, respiratory syncytial virus (RSV), influenza and coronavirus COVID-19 are all RNA viruses that cause respiratory infections and have error-prone RNA polymerases that enable frequent mutation.

- Human rhinovirus challenge in young adult allergic asthmatics (n=28) with healthy non-allergic controls (n=11): Nasosorption™ and Bronchosorption™ mucosal responses were described for viral load and a range of 30 cytokines and chemokines[12]. It is important to stress that much higher levels of mediators are detected in Nasosorption™ samples versus nasal lavage, and Bronchosorption™ versus broncho-alveolar lavage (BAL).
- Severe RSV bronchiolitis of infancy has been studied in terms of both nasal and bronchial inflammation, and interestingly infants with severe RSV infection causing respiratory failure have decreased levels of viral load and interferon[13,14]. In addition, nasal patterns of inflammation have been studied in relation to RSV experimental challenge in healthy adults: showing that differing patterns of neutrophilic inflammation contribute to RSV infection or protection from infection[15].
- Adults with severe influenza (SOIV, H1N1): including some with asthma have increased interferons in Nasosorption™ samples[16].
- Resiquimod (R848: TLR 7/8 agonist) given as a nasal challenge to induce a broad interferon (IFN) response[17], and this has been done without causing symptoms in asthma[18]. Resiquimod has potential as an immunostimulatory to boost IFN responses and can also be used to measure mucosal interferon responses in disease.
- More recently patterns of inflammation in Nasosorption™ samples have been studied in severe COVID-19 infection[19]. In addition, Nasosorption™ samples have been utilised to measure COVID-19 virus load, nasal IgG and IgA antibodies, and interferon and other cytokine responses both during infection with COVID-19 and following immunisation.

Bronchosorption™ has been employed in asthmatics before and during exacerbations in a human rhinovirus challenge model[12]. The Bronchosorption™ device has a central catheter to which a SAM is attached within an external catheter shaft. The Bronchosorption™ device is passed down the instrument channel of a bronchoscope, and when the tip of the catheter shaft is in the desired location, using a handpiece, the SAM is advanced to absorb a bronchial mucosal lining fluid sample. It has been demonstrated using samples from Nasosorption™ and Bronchosorption™ that nasal and bronchial mucosal cytokine responses are related after rhinovirus challenge (unpublished data), and there is also a correlation between Nasosorption™ IL-5 and sputum eosinophils in adult asthma[6].

However, nasopharyngeal and oropharyngeal sampling techniques, including Nasosorption™, have the disadvantage that, because the nasal mucociliary escalator (MCE) flows from the nares to the pharynx and is not continuous with the MCE from the lower airways through the bronchi and trachea, mucosal lining fluid from the back of the throat and the nasal passages may not contain the analytes and biomarkers which are present in mucosal lining fluid obtained from the lower respiratory tract or may be contaminated with materials which are not present in other parts of the respiratory tract, for example, saliva or material originating from the oesophagus.

Bronchial sampling provides a more accurate picture of what is taking place in the lungs and lower respiratory tract, but bronchial sampling techniques are typically more arduous for the patient, and are costly as compared with upper airway sampling. The procedures are generally carried out in an endoscopy suite by a specialist clinical team and the patient typically requires sedation, cardiorespiratory monitoring and local anaesthesia.

Conventional bronchial sampling techniques may only collect a relatively small volume of the available sample.

Furthermore, in the case of devices in which a sample is collected by a swab, there is a risk that fluid may be collected before the sampling device reaches the target sampling area. If a bronchial sampling device is passed through the operating channel of a bronchoscope, this may contain potentially contaminating fluids such as local anaesthetic, residual water from cleaning/-disinfection and secretions from the nose and upper airway, all of which may be prematurely absorbed by the swab.

Our previous application, WO 2019/239122 relates to a sampling device for obtaining samples of mucosal lining fluid which are expelled from the lower respiratory tract by forced expiration or coughing. This Coughsorption™ sampling device is placed in the oropharynx and is being developed for diagnosing and monitoring conditions such as eosinophilic asthma, pneumonia, chronic obstructive pulmonary disease, and tuberculosis. However, in some cases, more precise sampling of the airways beyond the larynx is required, for example for detection of inflammation, fibrosis, cancer biomarkers, metabolites, viruses, and pathogenic bacteria. It would be advantageous to be able to obtain mucosal samples from precise locations within the oesophagus or stomach or elsewhere in the gastrointestinal tract, and also to further reduce the risk of sample contamination by saliva or other substances present in the mouth or upper respiratory tract or gastrointestinal tract.

A further problem which may occur with conventional sampling devices is that materials used for swabs in the sampling devices have limited tensile strength and therefore there is a risk that, in use, the swab may become detached from the sampling device. Procedural control measures would be required to mitigate these risks.

Certain medical conditions affecting the upper gastrointestinal tract, for example, oesophageal cancer in patients with Barratt's oesophagus, eosinophilic oesophagitis, and gastro-oesophageal reflux disease (GERD) may also be diagnosed and monitored by sampling of mucosal lining fluid from the oesophagus.

Methods for oesophageal sampling include throat swabbing, endoscopic brushing and sampling using a Cytosponge™ device.

When obtaining samples of oesophageal mucosal lining fluid, similar problems may occur to the problems when obtaining samples from the respiratory tract. In particular, contamination of the sample with materials originating from the mouth, oropharynx and respiratory tract may occur.

Catheters for obtaining samples from the lower respiratory tract or upper gastrointestinal tract are known. For example, GB1511547 relates to a suction catheter for insertion into the nose, mouth or laryngotracheobroncheal tree. The suction catheter has an opening at its distal end and suffers from the known problems in obtaining an uncontaminated sample which are associated with such sampling devices. US 2017/0196547 relates to a suction catheter which can be inserted into the nose, nasopharynx or mouth and retracts the soft palate. The suction catheter is intended to remove accumulating fluid during surgery and is not optimal for obtaining samples of mucosal lining fluid. US 4,235,244 and US 4,329,995 relate to sampling catheters for obtaining uncontaminated specimens from the lower respiratory tract. The devices described in these documents are complex with a number of moving parts and it would be advantageous to provide a simpler device which is less likely to fail in use.

There is therefore a need for a device for taking samples of mucosal lining fluid from the trachea and bronchi or upper gastrointestinal tract, that is minimally invasive and which, in some cases, can avoid the need for bronchoscopy, oesophagoscopy or gastroscopy (endoscopy) by a clinical team in an endoscopy suite. It would be a major advantage to provide such a sampling device which can be used via a nasolaryngoscope at the bedside or in an outpatient clinical setting.

In conventional methods such as Bronchosorption™, a single sample of a volume of about 10-20 µL is typically obtained. It would be an advantage to provide a sampling device which makes it possible to obtain a larger sample of neat mucosal fluid and to regulate absorption speed and volume.

Furthermore, it would be advantageous if a sampling device could be left in place for a longer period of time compared to a Bronchosorption™ device, making it possible for a series of samples to be taken over a chosen period of time, and/or to take samples from different locations using the same sampling catheter.

WO 2013/084945 is considered the closest prior art to the invention, and discloses a suction catheter provided with a hollow catheter shaft and a liquid suction unit comprising a flexible, liquid-absorbent porous body provided to a distal end of the catheter shaft. The liquid suction unit comprises an outer layer and an inner layer and has an increasingly smaller pore diameter going inward in the layers, therefore adopting a structure where capillary action generates a force that moves liquid towards the catheter shaft side.

US 2021/244623 discloses a nasogastric feeding tube tip, which has a main body portion that fits to the end of a nasogastric or orogastric feeding tube. The main body has a hollow interior that is in fluid communication with the bore of the tube, and an aperture in a sidewall through which feed can be given to a patient, and through which a sample of stomach content can be aspirated. The outer wall of the main body comprises a recess or groove, which intersects the aperture, and which extends at least partially around the exterior surface of the main body.

US 5788680 is also relevant.

## Summary of the Invention

In a first aspect of the present invention, there is provided a sampling catheter as defined in claim 1.

Without pressure differential between the interior and exterior of the catheter of the present invention, the small apertures inhibit fluid ingress until the internal pressure of the catheter is reduced to below atmospheric pressure, drawing fluid and air within the catheter. The presence of the permeable material prevents air alone from entering the catheter, as airflow speed is reduced. This makes it possible for a reduced pressure to be maintained within the catheter behind the apertures so that the fluid and air can pass through. When the internal pressure of the catheter is returned to atmospheric pressure such that the internal and external pressures are again equalised, the small size of apertures again prevents the fluid from passing through, ensuring retention of the fluid sample within the catheter.

After the catheter is withdrawn, the fluid sample can be purged from the sample head by injecting buffer through the distal end of the catheter creating a washing rinsing action within the sampling head of the catheter, forcing fluid through the distal apertures into a collection vessel.

In some cases, the sampling region is in the respiratory tract. In this case, the sampling region is suitably in the lower airway, for example on the surface of the trachea or bronchi, particularly the trachea or one of the two primary bronchi. In this case, the sample site is chosen in order to obtain a sample which contains only material originating from the trachea or bronchi, but which is not contaminated with saliva or with material originating from the nose or pharynx.

Alternatively, the sampling region may be in the oesophagus. In this case, the sample site is chosen in order to obtain a sample which is not contaminated with saliva or with nasal or oropharyngeal contents. Sampling from the stomach and other parts of the gastrointestinal tract is also possible.

The catheter of the present invention has the advantage that it can be deployed via a nasolaryngoscope as well as *via* a bronchoscope or endoscope. Nasolaryngoscopy is a routine procedure in Ear Nose and Throat (ENT) clinics, and is generally employed by a single clinician, using a local anaesthetic spray for the nose and pharynx, and without sedation and cardiorespiratory monitoring. For example, the nasolaryngoscope may be positioned within the nose pharynx and larynx, allowing the vocal cords and oesophageal lumen to be directly visualised. With the nasolaryngoscope tip maintained above (or proximal to) the vocal cords, the catheter of the invention can then be advanced into the desired location(s) in the lower respiratory tract (airways distal to the vocal cords) or oesophagus and gastrointestinal tract for sampling.

Furthermore, it is possible to obtain a sample of neat mucosal fluid. This avoids the need for elution using an extraction buffer from a SAM. The volume of the sample may be known or can easily be determined so that biomarkers can be measured in terms of a known sample volume. This means that it is possible to determine the concentration of an analyte in mucosal fluid.

A further advantage is that there is a low risk that the permeable material may become detached from the catheter, unlike with some other sampling devices in which, as described above, a swab may become detached.

**Detailed Description of the Invention**

In the present specification, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention.

The catheter of the present invention is a sampling catheter. Therefore, in this specification, all references to a catheter refer to a sampling catheter.

In the present specification, the term "permeable material" refers to a material which is retained within the sampling head, and which is in contact with the exterior wall and with the apertures where they open into the interior cavity of the sampling head. The permeable material does not necessarily occupy the whole of the interior cavity of the sampling head but, because it covers the interior ends of the apertures, it is capable of ensuring that a negative pressure can build within the sampling head, even in cases where not all of the apertures are in contact with the sample fluid. This is described in more detail below.

Any type of permeable material can be used, provided that it is capable of functioning as just described. In some cases, the permeable material is a synthetic absorbent/adsorbent matrix.

In the present specification, the term "synthetic absorbent/adsorbent matrix (SAM)" refers to a matrix which has both absorbent and adsorbent properties. The SAM is capable of retaining some or all of the sample.

In the present specification, references to a sample fluid relate to a sample of mucosal lining fluid from the respiratory tract, especially the lower respiratory tract, or the upper gastrointestinal tract. The sample fluid comprises biomarkers which require detection and quantitation.

In the present specification, the term "diameter" has its usual meaning when referring to the diameter of a circle, e.g. a circular aperture or a catheter lumen of circular cross section. When referring to a shape which is not circular, e.g. a non-circular aperture, the term "diameter" refers to the greatest possible distance across the shape.

The proximal end of the sampling tube is suitable for connection to a suction device. Therefore, the suction catheter of the invention may comprise a suction device connected to the proximal end of the sampling tube. The suction device is adapted to reduce the pressure in the catheter lumen and the interior cavity of the sampling head so that a sample can be obtained as described in greater detail below. The suction device may be a pump, for example, a hand-operated or an electrically operated pump. Alternative suction devices include syringes. In some embodiments, the suction device may be capable of being operated in reverse such that the pressure inside the catheter lumen and sampling head is increased and the sample is expelled from the catheter.

In some embodiments, the catheter is a straight catheter with a flexible sampling tube.

In some cases, the sampling tube of a straight catheter may simply be inserted into the respiratory tract to the required depth and the sampling head is brought into contact with the mucosal lining fluid at the required sampling region.

Alternatively, a straight catheter may be inserted into the respiratory tract *via* the instrument operating channel of an endoscope. This is advantageous since the endoscope can be easily and accurately navigated to the sampling region using video or ultrasound. The endoscope can then be orientated into place and the sampling head of the catheter pushed into contact with the mucosal lining fluid of the respiratory tract at the target sampling region.

The sampling head and optionally all or part of the sampling tube of the catheter have a helical form. Catheters of this type may be formed from a thermoplastic material which is capable of holding a helical form. Suitably, the helical sampling head and sampling tube can be forced into a straight configuration but will adopt a helical configuration when the force is removed. Therefore, the helical catheter may comprise a non-helical outer sleeve surrounding the sampling head and sampling tube. In use, the outer sleeve may be inserted into the respiratory tract or oesophagus in order to position the sampling head at the required sampling region. In some embodiments, the sampling head and sampling tube are movable relative to the outer sleeve such that once the catheter is in position, the outer sleeve may be withdrawn, allowing the sampling head and optionally at least a part of the sampling tube to adopt a helical form, such that the outer edges of the helix contact the mucosal lining of the respiratory tract in the sampling region. Alternatively, the sampling head and optionally at least a part of the sampling tube may be pushed beyond the end of the outer sleeve such that they adopt a helical form as described above.

The external diameter of the sampling tube may be from about 1 mm to 6 mm, where diameters of 1 mm to 2 mm are preferred for narrow target sites such as the bronchi or where guided endoscopic manipulation is required, e.g., using a camera or ultrasound. For larger target sites, for example, the trachea or oesophagus, the diameter of the sampling tube is typically about 3 mm to 6 mm.

The internal diameter of the lumen of the sampling tube is suitably less than or equal to 1 mm, for example about 0.1 mm to 1.5 mm or 0.1 to 1.0 mm, more suitably less than or equal to 0.5 mm, for example about 0.1 mm to 0.5 mm.

The proximal end of the sampling tube, which is adapted to be attached to a pump or other device for reducing the internal pressure of the catheter, may be of a wider internal and/or external diameter for ease of connection to the device.

The diameter of the interior cavity of the sampling head may be considerably larger than that of the sampling tube. For example, the sampling head may be from 0.5 mm to 5.0 mm internal diameter and from 1.0 mm to 6.0 mm external diameter. A catheter having a sampling tube with a small external diameter and narrow lumen will usually have a sampling head with a small internal diameter, whereas, in a catheter with a larger diameter sampling tube will usually also have a sampling head with a larger diameter. In some embodiments, the sampling head is formed as a continuation of the sampling tube such that the external diameter is the same as that of the sampling tube, although the internal diameter may be larger.

In order for the sampling head of the catheter to be positioned at the target sampling region in the respiratory tract or upper gastrointestinal tract, the catheter may be from about 0.2 m to 3 m in length. The length comprises the total length of the sampling tube and the sampling head and will vary depending on the size of the patient and the design of the catheter. For example, a catheter intended for use with children will usually be shorter in length than one intended for use with adults. A catheter which is inserted via an endoscope will generally be longer than a straight catheter which is inserted directly into the airway or the oesophagus and a catheter with a helical sampling head and optionally a helical sampling tube will be longer still.

Suitably, the sampling head is from about 10 mm to 150 mm in length. In a catheter having a straight sampling tube, the sampling head is suitably about 10 mm to 50 mm in length, more suitably 25 mm to 45 mm or 30 mm to 40 mm long, and typically about 35 mm in length.

In a catheter having a helical sampling tube, the sampling head is suitably about 30 mm to 150 mm in length, more suitably 80 mm to 150 mm, still more suitably 100 mm to 140 mm or 110 mm to 130 mm and typically about 120 mm in length (where the length is measured when the sampling head is in a straight configuration within the outer sleeve).

The catheter of the present invention comprises a permeable material in the interior cavity of the sampling head. The permeable material may be formed from a foam or fibres. It is positioned within the sampling head such that it is in contact with the interior of the apertures and acts as a suppressive baffle, delaying the speed at which air passes through any non-occluded apertures and thus allowing sufficient negative pressure to build within the sampling head that the surface tension over occluded apertures is broken and the sample liquid enters the sampling head through these occluded apertures.

An important role of the permeable material is to allow fluid intake in cases where not all of the sampling head apertures are in contact with fluid sample, functioning as a suppressive baffle, delaying the speed at which pressure escapes, ensuring that when suction is applied to reduce the internal pressure of the catheter, the pressure difference between the interior cavity of the sampling head and the exterior of the cavity is maintained.

If there were no permeable material in the catheter, then if all of the apertures were to be in contact with a sample fluid, the sample fluid would enter the catheter through the apertures when the internal pressure of the catheter is reduced. However, in a case where there is no permeable material and a catheter has one or more non-occluded apertures, (i.e. apertures which are in contact with air rather than with a sample fluid) air will enter these non-occluded apertures, equalising any pressure differential between the interior and exterior of the catheter, further preventing the ingress of sample fluid into the sampling head, unless airspeed reaches critical flow rate, when fluid uptake is achievable.

In some cases, the permeable material may be formed from the same material used for swabs in conventional sampling devices. For example, the permeable material may be a synthetic absorbent/adsorbent matrix (SAM). The SAM is formed from a material which is capable of capturing and retaining at least part of a sample of mucosal lining fluid, including cells contained in the fluid, and is suitably in the form of a foam or a fibre matrix such that all or part of the sample is trapped within the foam or fibres.

More suitably, the material from which the SAM is formed releasably absorbs/adsorbs all or part of the sample of mucosal lining fluid such that the sample can be easily removed from the catheter for analysis, or that the sample is then passed into the lumen of the sampling tube and into a collection vessel, which may be connected to the proximal end of the sampling tube.

The permeable material, for example the SAM, may be formed from a breathable electrospun polymer such as polyester (PE), poly(lactic acid) (PLA), poly(lactic-co-glycolic acid) (PLGA), poly(methyl acrylate) (PMA) and $\varepsilon$-caprolactone. However, other suitable permeable materials, including SAMs, are known and are readily available.

Swab materials, including SAMs, have limited tensile strength therefore devices in which a swab makes direct contact with a sampling region within the patient have a potential risk of the swab becoming detached. Indeed, detachment of a swab has been known to occur with conventional sampling devices. The catheter of the invention has the advantage that the permeable material remains enclosed within the interior cavity (12) during sample collection and does not make direct contact with the sampling region within the patient so that the risk of detachment is prevented.

In many devices, swab materials only collect a relatively small volume of the available sample, for example, Bronchosorption™ with a 1 mm wide swab has a maximum fluid retention of not more than 20-60 $\mu$L. However, in the catheter of the present invention, the size of the sample is limited only by the capacity of the interior cavity (12), where the maximum sample size will generally correspond to the volume of the interior cavity (12). This means that it is possible to obtain much larger samples, for example, in some cases a sample having a volume of 100 $\mu$L to 1500 $\mu$L, more usually 150 $\mu$L to 1000 $\mu$L or 200 $\mu$L to 800 $\mu$L may be obtained.

In some embodiments, the sampling head is permanently attached to the sampling tube. In other embodiments, the sampling head is releasably attached to the sampling tube. In one embodiment, the catheter may be provided with a frangible ring which enables the sampling head to be removed from the catheter. Suitably, the frangible ring is positioned at the point where the sampling head is attached to the sampling tube such that the sampling head can simply be snapped away from the sampling tube.

In some embodiments where the sampling head is releasably attached to the sampling tube, the permeable material may comprise a handle for removing it from the sampling head once the sample has been obtained. The sample may then be extracted from the permeable material by any suitable means. These embodiments are particularly suitable when the permeable material is a SAM.

For example, the sample can be released from the permeable material (e.g. a SAM) by washing with an elution buffer. In embodiments where the sampling head is releasably attached to the sampling tube, the detached sampling head may be immersed in the elution buffer so that sample is released from the permeable material into the elution buffer, which is subsequently collected.

In embodiments where the sampling head is not releasably attached to the sampling tube, an elution buffer may be injected into the proximal end of the catheter and forced through the permeable material and out through the apertures of the sampling head into a collection vessel, carrying the sample with it.

In other embodiments where the sampling head is permanently attached to the sampling tube, the sample may be extracted by increasing the pressure inside the catheter so that the sample is forced out of the interior cavity, through the apertures in the sampling head and into an appropriate collection vessel.

Direct elution and washing of the sample is advantageous compared with more traditional methods in which the sample enters a swab and must then be removed using additional equipment such as extraction buffers and a vortex mixer or microcentrifuge. The advantages of the direct elution methods include reduced time and reduced risk of sample contamination. There is also generation of a neat sample of known volume and weight, initially undiluted in extraction buffer or fluids for assay, and thus biomarkers can be exactly quantitated in terms of the original fluid sample volume.

In some embodiments, the exterior wall of the sampling head has a rounded atraumatic tip for the comfort of the patient from whom a sample is to be obtained.

As already noted, the diameter of the apertures is substantially smaller than the diameters of the interior cavity of the sampling head and the lumen and is such that a sample fluid can pass through the apertures into the interior cavity only when the pressure in the interior cavity is reduced compared to the pressure outside the catheter. In some cases, the diameter of the apertures may be 100 to 1000 times smaller than the internal diameter of the cavity of the sampling head, suitably 100 to 600 times smaller or 100 to 400 times smaller and typically about 100 to 200 times smaller. The small size of the apertures ensures that when the pressure inside the catheter is the same as the external pressure, fluid outside the catheter forms a meniscus over the apertures and is therefore prevented from passing through the apertures into the catheter. This means that potentially contaminating fluids such as saliva, local anaesthetic, and unwanted material originating from the oesophagus cannot enter the catheter.

Once the catheter is positioned such that the sampling head is at the sampling region, the operator reduces the pressure inside the catheter lumen and the interior cavity such that it is lower than the external pressure. This can be achieved, for example by applying suction to the proximal end of the catheter. The reduced pressure allows the meniscus to be broken such that a sample fluid passes through the apertures into the interior cavity. As described in more detail below, the pressure reduction which is required will depend upon the size of the apertures and the surface tension of the sample liquid.

After the sample has been collected, the operator allows the pressure inside the catheter to equalise with the outside atmospheric pressure, and therefore there is no longer a pressure differential between the interior and exterior of the catheter. This can be achieved, for example, by ceasing suction at the proximal end of the catheter. Fluid is prevented from flowing out of the sampling head through the apertures because its surface tension causes it to form a droplet at each aperture on the exterior surface of the catheter. When the permeable material is a SAM, it collects cells as sample fluid flows through it.

The sampling head may comprise from about 10 to 500 apertures, more suitably about 50 to 500 apertures and typically about 100 to 400 apertures.

Suitably, the apertures are substantially circular in shape and have a diameter not greater than 100 $\mu$m, for example about 0.2 to 100 $\mu$m. The size of the apertures will depend upon the type of sample which is to be obtained.

In some cases, a sample will be required which contains all the components of mucosal lining fluid. In this case, there will be no filtering of cells or microorganisms from the sample and the apertures will suitably be from about 20 $\mu$m to 100 $\mu$m in diameter, more suitably from about 60 $\mu$m to 100 $\mu$m or 80 $\mu$m to 100 $\mu$m, for example about 90 $\mu$m in diameter.

In some samples, it is desirable to filter out large cells, particularly white blood cells, which are generally about 6 $\mu$m to 18 $\mu$m in diameter, and more particularly lymphocytes, neutrophils, basophils, eosinophils and monocytes. In this case, the apertures will be smaller, and will suitably be from about 5 $\mu$m to 11 $\mu$m in diameter, more suitably 7 $\mu$m to 9 $\mu$m, for example about 8 $\mu$m in diameter.

In other samples, it may be necessary to minimise the amounts of both white and red blood cells in the sample, while concentrating the amounts of bacteria and viruses. Red blood cells are generally about 6 $\mu$m to 8 $\mu$m in diameter so, for this purpose, the diameter of the apertures is suitably from about 1 $\mu$m to 5 $\mu$m, more suitably from 2 $\mu$m to 4 $\mu$m and typically about 2 $\mu$m.

In some samples, it may be desirable to remove cells, for example, bacterial cells or red and white blood cells, to obtain a soluble phase. This is the case where a cell-free or liquid biopsy is needed, for example when obtaining cell-free plasma for cancer biomarkers. Removal of bacteria may also be required when obtaining samples from patients with tuberculosis or suspected tuberculosis or to concentrate viruses in the sample. In such cases, apertures of a still smaller diameter may be required. Bacteria are generally from about 0.2 $\mu$m to 2 $\mu$m in size while viruses are about 0.02 $\mu$m to 0.5 $\mu$m, so for removal of bacteria, the apertures may be from about 0.02 $\mu$m to 0.5 $\mu$m in diameter, more suitably 0.05 $\mu$m to 0.25 $\mu$m, for example about 0.15 $\mu$m. If required, virus particles may also be removed from a sample using a device with the smallest apertures in this range.

The shape of the apertures may also be varied depending on the type of sample required and, in particular, on the properties of the required sample fluid and of potential contaminant fluids. In order to prevent the sample fluid from flowing out of the catheter through the apertures, the size of the aperture, the internal pressure in the catheter and the surface tension of the sample fluid must be such that the sample fluid forms a droplet over each of the apertures. Droplet formation is affected by factors such as aperture size and the surface tension, density and temperature of the sample fluid as well as the internal pressure of the catheter. Fluid retention is close to its maximum efficiency when droplets formed at the exterior of each aperture are hemispherical in shape. Reducing radial sphericity increases the head of fluid which can be retained within the catheter. The shape of the droplets may be affected by the configuration of the aperture. Therefore, in some cases, the aperture may be a simple opening, suitably a circular opening, in the wall of the sampling head of the catheter. Alternatively, the aperture may be a circular opening surrounded by an annular groove in the exterior wall of the sampling head. In a further alternative configuration, the aperture may be a circular opening surrounded by an annular groove in the exterior wall of the sampling head and both the circular opening and the annular groove are recessed into the exterior wall of the sampling head. In a variation of this configuration, the annular groove may be provided with bevelled edges.

In summary, the aperture may have one or more of the features:

i. the aperture is surrounded by an annular groove (31), (33) having a straight or bevelled edge.
ii. the aperture (20) is set in a recess (30) in the outer surface of the wall (14) of the sampling head.

The apertures may be arranged in any suitable configuration. In some cases, the apertures may be in rows on the sampling head, for example from 1 to 10 rows, 1 to 5 rows or 1 to 3 rows. The apertures may be arranged laterally along the surface of the sampling head and this configuration is particularly suitable when the sampling site is in a larger passage such as the trachea or oesophagus when the sample fluid is on the surface of the wall of the passage and therefore all or most of the laterally arranged apertures are in contact with the sample fluid. Alternatively, the apertures may be arranged around the whole surface of the sampling head, for example in a spiral pattern. More particularly, for optimal surface coverage, the apertures follow a biomimetic seed arrangement, arranged at the intersections of three families of spirals (phyllotaxis) of which are successive members of the Fibonacci sequence. This is particularly suitable for maximising fluid uptake of pooled fluids at the sampling site, as is the case where the sampling site is a smaller passage, for example the bronchi, or is the stomach.

The biomimetic seed positions of the apertures can be calculated in three stages, firstly by subdividing the radial profile of the sampling head by the number of apertures nodes n required, secondly recording the radial position from the axis r and the distance along the axis z of each node. Thirdly using the following conditional logic, calculate an angular direction $\theta$ about the axis for each node, where $\theta p$ is the previous angle in radians and $g$ is the golden angle of 2.4 radians.

$$\theta \begin{cases} \theta p + g - 2\pi & \theta p + g > 2\pi \\ \theta p + g & \theta p + g < 2\pi \end{cases}$$

If Cartesian co-ordinates for the aperture positions are required rather than the polar co-ordinates, the $\theta$ values can be changed to x and y values using the following:

$$x = r\ cos\ \theta$$

$$y = r\ sin\ \theta$$

These calculations enable the apertures to be positioned in the optimum position for the required use.

Further features of the catheter of the invention are described below with reference to the drawings.

## Description of the Drawings

Figure 1 is a cross-sectional view of an embodiment of a catheter according to the invention.

Figure 2 is a cross-sectional view of an alternative embodiment of a catheter according to the invention.

Figure 3 is a schematic view showing the positioning of a helical catheter according to the invention at a desired sampling location in the trachea of a patient, where the catheter is housed within a non-helical outer sleeve.

Figure 4 is a schematic view showing the use of an endoscope to position a catheter according to the invention at a desired sampling location in the bronchus of a patient.

Figure 5 is a schematic view showing apertures of different configurations at (I) to (IV).

## Description of the Embodiments of the Drawings

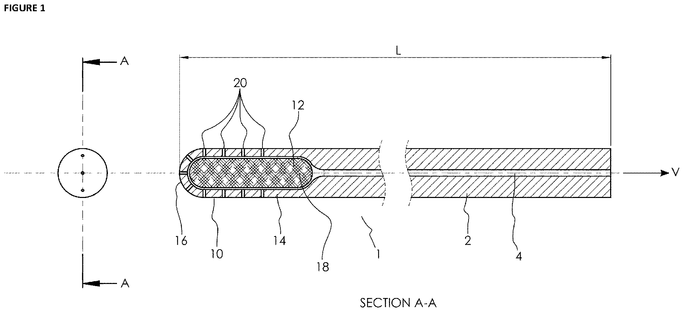

Figure 1 shows a suction catheter (1) according to the invention. The catheter (1) comprises a sampling tube (2) having a lumen (4) and a sampling head (10) at its distal end and is suitably attached at its proximal to a suction device represented by V. The catheter is suitably formed from a flexible plastic material such as high-density polyethylene (HDPE). The sampling tube (2) suitably has an external diameter of about 1.0 mm to 6.0 mm and the lumen (4) is suitably of a diameter of 0.1 mm to 1.5 mm, more suitably 0.1 mm to 0.5 mm. The catheter may comprise markings along the length of the sampling tube to assist an operator in positioning a sampling head (10) at the required sampling position in the airway or oesophagus.

The sampling head (10) may either be detachable or, alternatively, it may be formed as an integral part of the catheter (1).

The sampling head (10) of the catheter (1) is in fluid communication with the lumen (4) and is formed continuously with the sampling tube (2) such that the sampling tube (2) and sampling head (10) have the same external diameter. The sampling head has an inner cavity (12) defined by an exterior wall (14) which is closed at the tip (16) to prevent the entry of fluid from the oropharynx or oesophagus or the proximal airway when the sampling head is not positioned at the required sampling location. The tip (16) of the sampling head is atraumatic, i.e. it is rounded in order to minimise discomfort and potential injury to the patient. The diameter of the inner cavity (12) of the sampling head (10) is greater than that of the lumen of the sampling tube.

The exterior wall (14) of the sampling head (10) has apertures (20) which connect the inner cavity (12) with the exterior of the catheter. These apertures are of a diameter which is too small to allow ingress of fluid into the inner cavity (12) provided that there is no difference in pressure between the inner cavity and the exterior of the device.

Suitably, the sampling head comprises from about 10 to 500 apertures (20), more suitably about 50 to 500 apertures and typically about 100 to 400 apertures. The apertures are substantially circular in shape and suitably have a diameter not greater than 100 $\mu$m, for example about 0.2 $\mu$m to 100 $\mu$m. The number and size of the apertures will depend upon the type of sample to be obtained and the location of the sampling site. Suitable apertures can be manufactured using any suitable technique known to those of skill in the art, for example, micro drilling, laser methods, electron beam methods, ultrasonic vibration and micro hole punching techniques.

A permeable material (18) is positioned in the inner cavity (12) of the sampling head and is in contact with the inner ends of apertures (20) as shown in Figure 1.

The permeable material is suitably formed from a breathable electrospun polymer such as polyester (PE), poly(lactic acid) (PLA), poly(lactic-co-glycolic acid) (PLGA), poly(methyl acrylate) (PMA) and ε-caprolactone. The permeable material may be in the form of a foam or a fibre matrix and may be a SAM. As explained above, the material from which permeable material (18) is chosen so as to be suitable for regulating the difference in pressure between the interior cavity (12) and the exterior of the catheter: acting as a baffle across the catheter apertures (20). Thus, the permeable material (18) limits the speed at which air passes through any apertures which are not occluded by sample fluid and so allows sufficient negative pressure to build within the interior cavity (12) of the sampling head (10) that the surface tension over occluded apertures (20) is broken and the sample liquid enters the sampling head through these occluded apertures (20).

Suitably, the permeable material is a SAM and is also able to absorb and/or adsorb and retain all or part of the sample fluid together with cells contained in the sample fluid. It must also be able to release the sample efficiently when required.

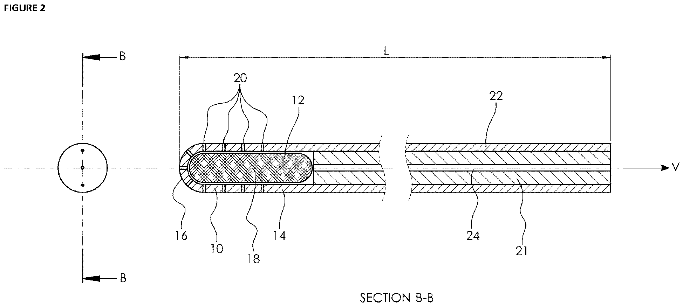

In the embodiment shown in Figure 2, the catheter sampling tube (21) having lumen (24) is positioned within an outer housing (22) which is continuous with the sampling head (10). The features of the sampling head are as described for the embodiment of Figure 1.

The external diameter of the outer housing (22) is similar to that of the sampling tube (2) of the embodiment of Figure 1 and the internal diameter of the lumen (24) of the sampling tube (21) is similar to that of lumen (4) of the sampling tube (2) of Figure 1.

The embodiment of Figure 2 has the advantage that the same outer housing (22) could be used with sampling tubes (21) having lumens (24) of different diameters. In addition, when assembling the catheter of Figure 2, the sampling tube (21) can be used to position the permeable material (18) in the sampling head (10). In some embodiments, the permeable material (18) may be attached to the sampling tube (21) and, in this case, the sampling tube (21) can be used as a handle to remove the permeable material (18) from the sampling head once the sample has been collected.

In use, the catheter (1) is inserted *via* the mouth or nose into the airway or the oesophagus of a patient and the sampling head (10) is positioned such that it is in contact with the mucosal lining of the airway or upper gastrointestinal tract at a desired sampling position. The positioning of the sampling head (10) at the required sampling position may be assisted by the presence of markings on the outer surface of the sampling tube (2). In some cases, the catheter (1) may comprise an actuator which moves the sampling head (10) into position but more suitably, the sampling head (10) may be of a design such that this is not necessary.

When the sampling head (10) is in contact with the mucosal lining of the airway or upper gastrointestinal tract, some or all of the apertures (20) are occluded by the mucosal lining fluid. The mucosal lining fluid does not enter the device because the surface tension is too great to allow it to pass through the apertures (20). The operator then uses the suction device to reduce the pressure in the interior of the catheter. When the pressure in the interior of the catheter is lower than the pressure outside the catheter, the mucosal lining fluid is able to pass through the apertures (20) into the inner cavity (12) and flows through the permeable material (18).

The pressure differential required for the mucosal lining fluid to pass through the apertures (20) depends on several factors, the most significant of which is the size of the apertures (20). In general, assuming that the bronchial mucous has a density of about 1500 kg/m$^3$ and a surface tension of about 0.03 N/m, and that the apertures are about 0.2 to 100 μm in diameter, the pressure differential is suitably from about 1200 Pa and 60000 Pa due to variation in aperture geometry, material property and the environmental conditions. For this range of pressure difference, at sea level, where atmospheric pressure is 101325 Pa, a vacuum of from 100125 Pa (to give a 1200 Pa pressure difference) down to 41325 Pa (to give a 60000 Pa pressure difference) would be required.

The suction device indicated by V may be a pump, which may be hand operated or electrically operated. In some cases, a syringe may be used as a suction device.

When the sample has been collected, the catheter (1) is withdrawn from the patient. In some cases, the sample may be removed from the device by inserting the sampling tip into a collection vessel and then applying a positive pressure to the catheter (1) such that the pressure in the inner cavity (12) of the sampling head is higher than the pressure on the exterior of the device and the sample passes from the permeable material through the apertures and into the collection vessel. The positive pressure can be applied via the suction device. In some cases, an elution buffer may be injected into the proximal end of the catheter and then forced through the permeable material (18) in the sampling head and out through the apertures (20) carrying with it the sample from the inner cavity (12), including any sample which has been absorbed and/or adsorbed by the permeable material (especially when the permeable material is a SAM).

Alternatively, in the embodiment of Figure 2, the sampling tube (21) may be attached to the permeable material (18) and may be used to remove the permeable material (18) from the sampling head (10). In embodiments where the permeable material is removed from the device, it is preferred that the permeable material is a SAM, since this will absorb and/or adsorb most or all of the sample including cells contained in the sample liquid.

In embodiments where the sampling head (10) is detachable, the permeable material (e.g. a SAM) may be provided with a handle which can be used to remove the samplecontaining permeable material (e.g. SAM) from the sampling head. In embodiments where the permeable material (e.g. SAM) is removed from the device, it may be washed with or immersed in an elution buffer and, if necessary, compressed after removal from the buffer to release the sample.

The catheter may be inserted into the airway or oesophagus of a patient *via* the mouth or nose.

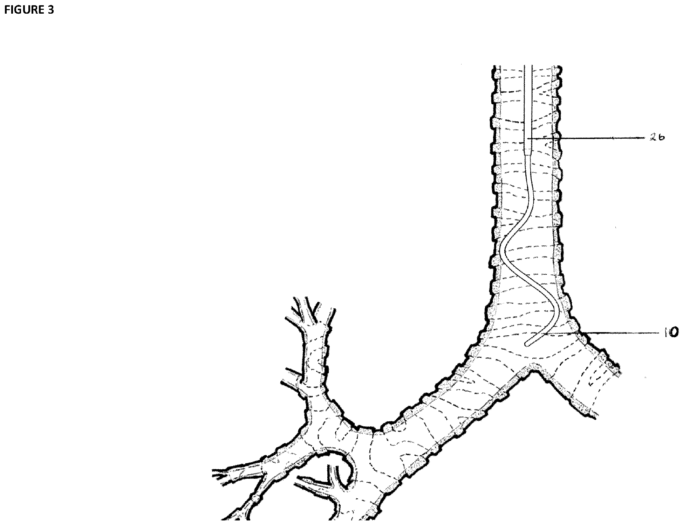

Figure 3 shows a helical catheter of the invention inserted into the trachea. The sampling head (10) is in helical form and is formed from thermoplastic polymers such as High-Density Polyethylene (HDPE) or Polypropylene (PP), and thermoplastic elastomers such as Pebax® or Santoprene. The sampling tube (2) and head (10) are fitted inside a non-helical outer sleeve (26) for ease of insertion into the trachea of the patient.

In use, the housed catheter assembly is inserted into the oral cavity or the nose towards the target site. As with the device of Figure 1, the catheter may comprise markings to assist with positioning the sampling head (10) at the required sampling site. However, in the device of Figure 3, the markings may be on the outer sleeve (26). Alternatively, a clinician may be able to position the device using feedback from known anatomical locations. For example, when the device is inserted into the trachea, the carina may be used for this purpose. Once the sampling head (10) is in position, the outer sleeve (26) may be withdrawn such that the sampling head (10) adopts a helical configuration at the sampling site. As noted above, the sampling head (10) of the helical catheter may be from about 30 mm to 150 mm in length but is typically about 120 mm in length (where the length is measured when the sampling head is in a straight configuration within the outer sleeve).

A sample is obtained by reducing the internal pressure in the sampling tube (2) and sampling head (10) as described above. In order to withdraw the catheter from the patient, the outer sleeve may be extended again so that it covers the sampling head (10), forcing the catheter into a straight configuration once more.

In a variation of the embodiment of Figure 3, the catheter also comprises an actuator which can be operated to move the sampling head (10) beyond the end of the outer sleeve (26) to the sampling location. Once a sample has been obtained, the sampling head (10) is withdrawn into the outer sleeve (26) by means of the actuator and the outer sleeve (26) is withdrawn via the mouth or nose so that the sample can be removed from the catheter.

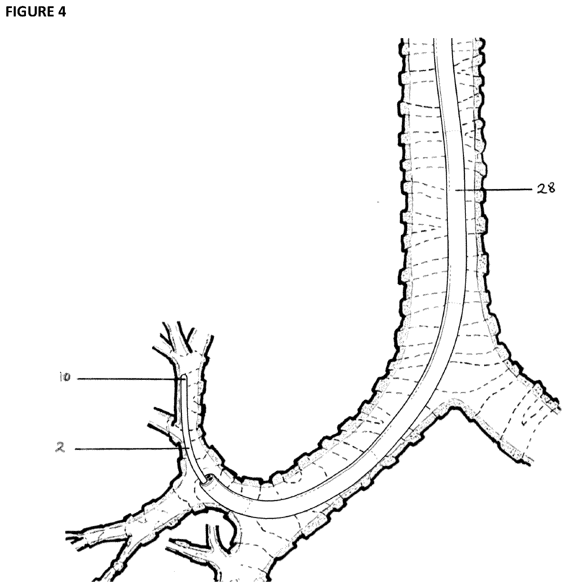

In a further alternative embodiment shown in Figure 4, the catheter (1) can be inserted into the respiratory tract with the help of an endoscope (28). In this case, the endoscope (28) is inserted into the airway of a patient via the mouth or nose and the sample tube (2) of the catheter (1) is passed through the lumen (instrument channel) of the endoscope (28) and into the airway until the sampling head (10) is positioned at the required sampling region. In Figure 4, the sampling region is one of the bronchi. The endoscope (28) may be navigated to the target region by means of video or ultrasonic methods, which are well known in the art. Again, similar apparatus can be used to insert the catheter of the invention into the upper gastrointestinal tract of a patient.

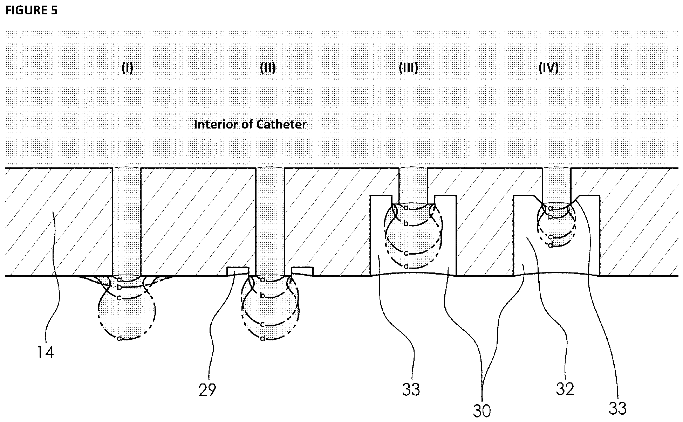

Figure 5 shows how the design of the apertures (20) can be adapted to retain a sample within the sampling head for different types of fluid sample. The figure shows the wall (14) of the sampling head and illustrates circular apertures of different designs at (I), (II), (III) and (IV). For each of these aperture designs, the letters a, b, c and d illustrate the shape of a droplet forming at the outside of the aperture as the pressure within the catheter increases, with (a) representing the lowest pressure and (d) representing the highest pressure.

The maximum height of fluid which can be retained in the catheter can be calculated using the equation:

$$h = \frac{2\gamma}{\rho g r}$$

where

h is the maximum height of fluid which can be retained in the catheter (m);
$\gamma$ is the surface tension of the sample fluid (Nm$^{-1}$);
$\rho$ is the density of the sample fluid (kgm$^{-3}$);
g is the gravitational acceleration (ms$^{-2}$);
r is the droplet radius (m).

For optimal retention of the sample fluid within the catheter, the droplet should be hemispherical in shape, as represented by (a) in each of the designs (I), (II), (III) and (IV). Droplet formation is affected not only by the pressure of the liquid inside the catheter but also by the size of the aperture, the surface tension between the sample fluid and the material from which the catheter is made, and the density, viscosity, mucus content and temperature of the sample fluid.

The aperture shown at (I) is a simple circular hole in the wall (14) of the catheter and the droplet shown at (a) is substantially hemispherical in shape, meaning that the head of fluid which can be retained in the catheter is at a maximum. An increase of pressure inside the catheter changes the form of the droplet to that shown at (b), in which the droplet has spread across the outer surface of the catheter wall (14), wetting its surface. Since the height of fluid which can be retained in the catheter is inversely proportional to the radius of the droplet, this reduces the head of fluid which can be retained in the catheter. As the pressure increases further the surface tension in the fluid pulls the droplet back towards the hole as shown by (c) and (d).

The variant shown at (II) has an annular groove (29) surrounding the circular aperture (20). The wall of the annular groove (29) limits the spread of the droplet at (b) and (c) such that the retention height is increased.

The variants shown at (III) and (IV) are intended to overcome potential problems caused by the surface of the catheter coming into contact with the wall of the trachea or oesophagus, particularly when the catheter is withdrawn, such that the droplets are removed from the surface of the sampling head, breaking the surface tension and causing the sample to leak from the catheter. Similar problems can arise for a helical catheter which has an outer sleeve or for a catheter used with an endoscope. In each of the variants shown at (III) and (IV), the aperture is set in a recess (30) in the outer surface of the wall (14) of the sampling head.

The variant shown at (III) is similar to that shown at (II) in that the aperture (20) is surrounded by an annular groove (31), which limits the spread of the droplet when the internal pressure of the catheter is increased.

In the variant shown at (IV), the aperture (20) is also surrounded by an annular groove (33), but in this case the groove (33) has a bevelled edge which further reduces the diameter of the droplet so that it is the same as that of the bore of the aperture (20), meaning that the retention height in the catheter is further increased.

It is therefore possible by varying the configuration of the apertures (20) in the sampling head (2) to ensure that the sample is retained in the sampling head once it has been obtained and for the time taken to remove the catheter from the patient. After removal of the catheter, the sample can be removed from the catheter as described above.

The invention in its various embodiments provides a means for obtaining a sample of mucosal lining fluid from the airway, especially the upper and lower airway, or the upper gastrointestinal tract of a patient.

**References**

1. Jha A, Thwaites RS, Singh N, Hansel TT. Chapter 8.1 Precision Mucosal Sampling and Biomarkers in Allergic Rhinitis and Asthma. Carini, C., Fidock, M., & van Gool, A. (Eds.). (2019). "Handbook of Biomarkers and Precision Medicine" (1st ed.). Chapman and Hall/CRC; 2019.

2. Thwaites RS, Jarvis HC, Singh N, Jha A, Pritchard A, Fan H, Tunstall T, Nanan J, Nadel S, Kon OM, Openshaw PJ, Hansel TT. Absorption of Nasal and Bronchial Fluids: Precision Sampling of the Human Respiratory Mucosa and Laboratory Processing of Samples. *J Vis Exp 2018.*

3. Chawes BL, Edwards MJ, Shamji B, Walker C, Nicholson GC, Tan AJ, Folsgaard NV, Bonnelykke K, Bisgaard H, Hansel TT. A novel method for assessing unchallenged levels of mediators in nasal epithelial lining fluid. J Allergy Clin Immunol 2010; 125: 1387-1389 e1383.

4. Folsgaard NV, Chawes BL, Rasmussen MA, Bischoff AL, Carson CG, Stokholm J, Pedersen L, Hansel TT, Bonnelykke K, Brix S, Bisgaard H. Neonatal cytokine profile in the airway mucosal lining fluid is skewed by maternal atopy. Am J Respir Crit Care Med 2012; 185: 275-280.

5. Wolsk HM, Folsgaard NV, Birch S, Brix S, Hansel TT, Johnston SL, Kebadze T, Chawes BL, Bonnelykke K, Bisgaard H. Picornavirus-Induced Airway Mucosa Immune Profile in Asymptomatic Neonates. J Infect Dis 2016; 213: 1262-1270.

6. Melo JT, Jr., Tunstall T, Pizzichini MMM, Maurici R, Rocha CC, Dal-Pizzol F, Goncalves J, Hansel TT, Thwaites RS, Pizzichini E. IL-5 Levels in Nasosorption and Sputosorption Correlate with Sputum Eosinophilia in Allergic Asthma. Am J Respir Crit Care Med 2019; 199: 240-243.

7. Scadding GW, Calderon MA, Bellido V, Koed GK, Nielsen NC, Lund K, Togias A, Phippard D, Turka LA, Hansel TT, Durham SR, Wurtzen PA. Optimisation of grass pollen nasal allergen challenge for assessment of clinical and immunological outcomes. J Immunol Methods 2012; 384: 25-32.

8. Leaker BR, Malkov VA, Mogg R, Ruddy MK, Nicholson GC, Tan AJ, Tribouley C, Chen G, De Lepeleire I, Calder NA, Chung H, Lavender P, Carayannopoulos LN, Hansel TT. The nasal mucosal late allergic reaction to grass pollen involves type 2 inflammation (IL-5 and IL-13), the inflammasome (IL-1beta), and complement. Mucosal Immunol 2017; 10: 408-420.

9. Thwaites RS, Gunawardana NC, Broich V, Mann EH, Ahnstrom J, Campbell GA, Lindsley S, Singh N, Tunstall T, Lane DA, Openshaw PJ, Hawrylowicz CM, Hansel TT. Biphasic activation of complement and fibrinolysis during the human nasal allergic response. J Allergy Clin Immunol 2018; 141: 1892-1895 e1896.

10. Nicholson GC, Kariyawasam HH, Tan AJ, Hohlfeld JM, Quinn D, Walker C, Rodman D, Westwick J, Jurcevic S, Kon OM, Barnes PJ, Krug N, Hansel TT. The effects of an anti-IL-13 mAb on cytokine levels and nasal symptoms following nasal allergen challenge. J Allergy Clin Immunol 2011; 128: 800-807 e809.

11. Dhariwal J, Kitson J, Jones RE, Nicholson G, Tunstall T, Walton RP, Francombe G, Gilbert J, Tan AJ, Murdoch R, Kon OM, Openshaw PJ, Hansel TT. Nasal Lipopolysaccharide Challenge and Cytokine Measurement Reflects Innate Mucosal Immune Responsiveness. PLoS One 2015; 10: e0135363.

12. Hansel TT, Tunstall T, Trujillo-Torralbo MB, Shamji B, Del-Rosario A, Dhariwal J, Kirk PDW, Stumpf MPH, Koopmann J, Telcian A, Aniscenko J, Gogsadze L, Bakhsoliani E, Stanciu L, Bartlett N, Edwards M, Walton R, Mallia P, Hunt TM, Hunt TL, Hunt DG, Westwick J, Edwards M, Kon OM, Jackson DJ, Johnston SL. A Comprehensive Evaluation of Nasal and Bronchial Cytokines and Chemokines Following Experimental Rhinovirus Infection in Allergic Asthma: Increased Interferons (IFN-gamma and IFN-lambda) and Type 2 Inflammation (IL-5 and IL-13). EBioMedicine 2017; 19: 128-138.

13. Thwaites RS, Ito K, Chingono JMS, Coates M, Jarvis HC, Tunstall T, Anderson-Dring L, Cass L, Rapeport G, Openshaw PJ, Nadel S, Hansel TT. Nasosorption as a Minimally Invasive Sampling Procedure: Mucosal Viral Load and Inflammation in Primary RSV Bronchiolitis. J Infect Dis 2017; 215: 1240-1244.

14. Thwaites RS, Coates M, Ito K, Ghazaly M, Feather C, Abdulla F, Tunstall T, Jain P, Cass L, Rapeport G, Hansel TT, Nadel S, Openshaw P. Reduced Nasal Viral Load and IFN Responses in Infants with Respiratory Syncytial Virus Bronchiolitis and Respiratory Failure. Am J Respir Crit Care Med 2018; 198: 1074-1084.

15. Habibi MS, Thwaites RS, Chang M, Jozwik A, Paras A, Kirsebom F, Varese A, Owen A, Cuthbertson L, James P, Tunstall T, Nickle D, Hansel TT, Moffatt MF, Johansson C, Chiu C, Openshaw PJM. Neutrophilic inflammation in the respiratory mucosa predisposes to RSV infection. Science 2020; 370.

16. Jha A, Dunning J, Tunstall T, Thwaites RS, Hoang LT, Investigators M, Kon OM, Zambon MC, Hansel TT, Openshaw PJ. Patterns of systemic and local inflammation in patients with asthma hospitalised with influenza. Eur Respir J 2019; 54.

17. Progatzky F, Jha A, Wane M, Thwaites RS, Makris S, Shattock RJ, Johansson C, Openshaw PJ, Bugeon L, Hansel TT, Dallman MJ. Induction of innate cytokine responses by respiratory mucosal challenge with R848 in zebrafish, mice, and humans. J Allergy Clin Immunol 2019; 144: 342-345 e347.

18. Jha A, Thwaites RS, Tunstall T, Kon OM, Shattock RJ, Hansel TT, Openshaw PJM. Increased nasal mucosal interferon and CCL13 response to a TLR7/8 agonist in asthma and allergic rhinitis. J Allergy Clin Immunol 2021; 147: 694-703 e612.

19. Thwaites RS, Sanchez Sevilla Uruchurtu A, Siggins MK, Liew F, Russell CD, Moore SC, Fairfield C, Carter E, Abrams S, Short CE, Thaventhiran T, Bergstrom E, Gardener Z, Ascough S, Chiu C, Docherty AB, Hunt D, Crow YJ, Solomon T, Taylor GP, Turtle L, Harrison EM, Dunning J, Semple MG, Baillie JK, Openshaw PJ, investigators IC. Inflammatory profiles across the spectrum of disease reveal a distinct role for GM-CSF in severe COVID-19. Sci Immunol 2021; 6.

**Claims**

1. A catheter (1) for obtaining a sample of mucosal lining fluid from a sampling region in the respiratory tract or the upper gastrointestinal tract, wherein the catheter comprises:

    i. a sampling tube (2) having a lumen (4); and
    ii. a sampling head (10) at the distal end of the sampling tube, wherein the sampling head comprises an interior cavity (12) in fluid connection with the lumen and a permeable material (18) within the interior cavity;

    wherein the sampling head is closed by an exterior wall (14) in which there are a plurality of apertures (20), wherein the permeable material is in contact with the exterior wall and the apertures and wherein the diameter of the apertures is substantially smaller than the internal diameter of the lumen and is such that a sample fluid can pass through the apertures into the interior cavity only when the pressure in the interior cavity is reduced compared to the pressure outside the catheter;
    **characterised in that** the sampling head (10) has a helical form.

2. A catheter (1) according to claim 1, further comprising a suction device (V) connected to the proximal end of the sampling tube.

3. A catheter (1) according to claim 1 or claim 2, wherein:

    the external diameter of the sampling tube (2) is from 1 mm to 6 mm; and/or
    the internal diameter of the catheter lumen (4) is from 0.1 mm to 1.5 mm; and/or
    the internal diameter of the sampling head (10) is from 0.5 mm to 5 mm; and/or
    the catheter is from 0.2 m to 3 m in length; and/or
    the sampling tube (2) is substantially straight and is optionally adapted to be inserted via the instrument operating channel of an endoscope.

4. A catheter (1) according to any one of claims 1 to 3, wherein all or part of the sampling tube (2) has a helical form.

5. A catheter (1) according to any one of claims 1 to 4, further comprising a non-helical outer sleeve (26) surrounding the sampling head (10) and sampling tube (2).

6. A catheter (1) according to claim 5, wherein the sampling tube (2) and sampling head (10) are movable relative to the outer sleeve.

7. A catheter (1) according to any one of claims 1 to 6, wherein the sampling head (10) is from 30 mm to 150 mm in length.

8. A catheter (1) according to any one of claims 1 to 7, wherein the permeable material (18) is formed from an electrospun polymer such as polyester (PE), poly(lactic acid) (PLA), poly(lactic-co-glycolic acid) (PLGA), poly(methyl acrylate) (PMA) and ε-caprolactone.

9. A catheter (1) according to any one of claims 1 to 8, wherein the permeable material (18) is a SAM.

10. A catheter (1) according to any one of claims 1 to 9, wherein the sampling head (10) is releasably attached to the sampling tube (2).

11. A catheter (1) according to any one of claims 1 to 10, wherein the sampling head (10) comprises 10 to 500 apertures (20).

12. A catheter (1) according to any one of claims 1 to 11, wherein the apertures (20) are circular and have a diameter of 0.2 μm to 100 μm.

13. A catheter (1) according to claim 12, wherein the apertures (20) have one or more of the features:

    i. the aperture is surrounded by an annular groove (29) having a straight or bevelled edge;
    ii. the aperture is set in a recess (30) in the outer surface of the wall (14) of the sampling head (10).

14. A catheter (1) according to any one of claims 1 to 13, wherein the apertures (20) are arranged in from 1 to 10 rows on the sampling head (10).

15. A catheter (1) according to any one of claims 1 to 14, wherein the apertures (20) are arranged around the whole surface of the sampling head (10), for example in a spiral pattern.

**Patentansprüche**

1. Katheter (1) zum Erhalten einer Probe eines Darmschleimhautfluids aus einem Entnahmebereich im Atemtrakt oder im oberen Magen-Darm-Trakt, wobei der Katheter Folgendes umfasst:

    i. ein Probenahmeröhrchen (2), das ein Lumen (4) aufweist; und
    ii. einen Probenahmekopf (10) am distalen Ende des Probenahmeröhrchens, wobei der Probenahmekopf einen inneren Hohlraum (12) in Fluidverbindung mit dem Lumen und ein durchlässiges Material (18) innerhalb des inneren Hohlraums umfasst;
    wobei der Probenahmekopf durch eine Außenwand (14) abgeschlossen ist, in der eine Vielzahl von Öffnungen (20) vorhanden sind, wobei das durchlässige Material in Kontakt mit der Außenwand und den Öffnungen steht und wobei der Durchmesser der Öffnungen im Wesentlichen kleiner als der Innendurchmesser des Lumens und derart beschaffen ist, dass ein Probefluid nur durch die Öffnungen in den inneren Hohlraum passieren kann, wenn der Druck im inneren Hohlraum im Vergleich zum Druck außerhalb des Katheters reduziert ist;
    **dadurch gekennzeichnet, dass** der Probenahmekopf (10) eine spiralförmige Form aufweist.

2. Katheter (1) nach Anspruch 1, der ferner eine Saugvorrichtung (V) umfasst, die mit dem proximalen Ende des Probenahmeröhrchens verbunden ist.

3. Katheter (1) nach Anspruch 1 oder Anspruch 2, wobei:

    der Außendurchmesser des Probenahmeröhrchens (2) zwischen 1 mm und 6 mm beträgt; und/oder
    der Innendurchmesser des Katheterlumens (4) zwischen 0,1 mm und 1,5 mm beträgt; und/oder
    der Innendurchmesser des Probenahmekopfs (10) zwischen 0,5 mm und 5 mm beträgt; und/oder
    die Länge des Katheters zwischen 0,2 m und 3 m beträgt; und/oder
    der Probenahmekopf (2) im Wesentlichen gerade und optional so ausgelegt ist, dass er über den Instrumentenoperationskanal eines Endoskops eingeführt werden kann.

4. Katheter (1) nach einem der Ansprüche 1 bis 3, wobei das ganze Probenahmeröhrchen (2) oder ein Teil davon eine spiralförmige Form aufweist.

5. Katheter (1) nach einem der Ansprüche 1 bis 4, der ferner eine nichtspiralförmige Außenhülse (26) umfasst, die den Probenahmekopf (10) und das Probenahmeröhrchen (2) umgibt.

6. Katheter (1) nach Anspruch 5, wobei das Probenahmeröhrchen (2) und der Probenahmekopf (10) in Bezug auf die Außenhülse beweglich sind.

7. Katheter (1) nach einem der Ansprüche 1 bis 6, wobei die Länge des Probenahmekopfs (10) zwischen 30 mm und 150 mm beträgt.

8. Katheter (1) nach einem der Ansprüche 1 bis 7, wobei das durchlässige Material (18) aus einem elektrogesponnenen Polymer wie Polyester (PE), Poly(milchsäure) (PLA), Poly(milchsäure-coglykolsäure) (PLGA), Poly(methylacrylat) (PMA) und ε-Caprolacton gebildet ist.

9. Katheter (1) nach einem der Ansprüche 1 bis 8, wobei das durchlässige Material (18) ein SAM ist.

10. Katheter (1) nach einem der Ansprüche 1 bis 9, wobei der Probenahmekopf (10) entfernbar an dem Probenahmeröhrchen (2) befestigt ist.

11. Katheter (1) nach einem der Ansprüche 1 bis 10, wobei der Probenahmekopf (10) 10 bis 500 Öffnungen (20) umfasst.

12. Katheter (1) nach einem der Ansprüche 1 bis 11, wobei die Öffnungen (20) kreisförmig sind und einen Durchmesser zwischen 0,2 μm und 100 μm aufweisen.

13. Katheter (1) nach Anspruch 12, wobei die Öffnungen (20) eines oder mehrere der folgenden Merkmale aufweisen:

    i. die Öffnung ist von einer ringförmigen Nut (29) mit einer geraden oder abgeschrägten Kante umgeben;
    ii .die Öffnung ist in einer Vertiefung (30) in der Außenfläche der Wand (14) des Probenahmekopfs (10) eingerichtet.

14. Katheter (1) nach einem der Ansprüche 1 bis 13, wobei die Öffnungen (20) in 1 bis 10 Reihen am Probenahmekopf (10) angeordnet sind.

15. Katheter (1) nach einem der Ansprüche 1 bis 14, wobei die Öffnungen (20) um die gesamte Fläche des Probenahmekopfs (10) herum, beispielsweise in einem spiralförmigen Muster, angeordnet sind.

**Revendications**

1. Cathéter (1) permettant d'obtenir un échantillon de fluide de revêtement muqueux depuis une zone d'échantillonnage dans le tractus respiratoire du tractus gastrointestinal supérieur, le cathéter comprenant :

    i. un tube d'échantillonnage (2) doté d'un lumen (4) ; et
    ii. une tête d'échantillonnage (10) à l'extrémité distale du tube d'échantillonnage, la tête d'échantillonnage comprenant une cavité intérieure (12) en connexion fluidique avec le lumen et un matériau perméable (18) dans la cavité intérieure ;
    la tête d'échantillonnage étant fermée par une paroi extérieure (14) dans laquelle il y a une pluralité d'ouvertures (20), le matériau perméable étant en contact avec la paroi extérieure et les ouvertures et le diamètre des ouvertures étant substantiellement inférieur au diamètre intérieur du lumen et étant tel qu'un fluide d'échantillonnage puisse passer à travers les ouvertures jusque dans la cavité intérieure seulement si la pression dans la cavité intérieure est réduite comparativement à la pression à l'extérieur du cathéter ;
    **caractérisé en ce que** la tête d'échantillonnage (10) a une forme hélicoïdale.

2. Cathéter (1) selon la revendication 1, comprenant en outre un dispositif d'aspiration (V) connecté à l'extrémité proximale du tube d'échantillonnage.

3. Cathéter (1) selon la revendication 1 ou la revendication 2, dans lequel :

    le diamètre extérieur du tube d'échantillonnage (2) va de 1 mm à 6 mm ; et/ou
    le diamètre intérieur du lumen du cathéter (4) va de 0,1 mm à 1,5 mm ; et/ou
    le diamètre intérieur de la tête d'échantillonnage (10) va de 0,5 mm à 5 mm ; et/ou
    le cathéter a une longueur de 0,2 à 3 m ; et/ou
    le tube d'échantillonnage (2) est sensiblement droit et est en option apte à être inséré via le canal d'actionnement d'instrument d'un endoscope.

4. Cathéter (1) selon l'une quelconque des revendications 1 à 3, dans lequel tout ou partie du tube d'échantillonnage (2) a une forme hélicoïdale.

5. Cathéter (1) selon l'une quelconque des revendications 1 à 4, comprenant en outre un manchon extérieur non hélicoïdal (26) entourant la tête d'échantillonnage (10) et le tube d'échantillonnage (2).

**6.** Cathéter (1) selon la revendication 1 ou la revendication 5, dans lequel le tube d'échantillonnage (2) et la tête d'échantillonnage (10) sont mobiles par rapport au manchon extérieur.

**7.** Cathéter (1) selon l'une quelconque des revendications 1 à 6, dans lequella tête d'échantillonnage (10) a une longueur de 30 mm à 150 mm.

**8.** Cathéter (1) selon l'une quelconque des revendications 1 à 7, dans lequel le matériau perméable (18) est formé d'un polymère électrofilé comme du polyester (PE), de l'acide polylactique (PLA), de l'acide polylactique-coglycolique (PLGA), du poly(méthylmétacrylate) (PMA) et du έ-caprolactone.

**9.** Cathéter (1) selon l'une quelconque des revendications 1 à 8, dans lequel le matériau perméable (18) est un SAM.

**10.** Cathéter (1) selon l'une quelconque des revendications 1 à 9, dans lequel la tête d'échantillonnage (10) est fixée de manière amovible au tube d'échantillonnage (2).

**11.** Cathéter (1) selon l'une quelconque des revendications 1 à 10, dans lequel la tête d'échantillonnage (10) comprend 10 à 500 ouvertures (20).

**12.** Cathéter (1) selon l'une quelconque des revendications 1 à 11, dans lequel les ouvertures (20) sont circulaires et ont un diamètre de 0,2 $\mu$m à 100 $\mu$m.

**13.** Cathéter (1) selon la revendication 12, dans lequel les ouvertures (20) ont une ou plusieurs des caractéristiques suivantes :

    i. l'ouverture est entourée d'une gorge annulaire (20) ayant un bord droit ou biseauté ;
    ii. l'ouverture est placée dans un retrait (30) de la surface extérieure de la paroi (14) de la tête d'échantillonnage (10).

**14.** Cathéter (1) selon l'une quelconque des revendications 1 à 13, dans lequel les ouvertures (20) sont disposées dans 1 à 10 rangées sur la tête d'échantillonnage (10).

**15.** Cathéter (1) selon l'une quelconque des revendications 1 à 14, dans lequel les ouvertures (20) sont disposées autour de l'ensemble de la surface de la tête d'échantillonnage (10), par exemple suivant un motif en spirale.

**FIGURE 1**

SECTION A-A

**FIGURE 2**

SECTION B-B

FIGURE 3

**FIGURE 4**

**FIGURE 5**

**EP 4 289 369 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019239122 A
- GB 1511547 A
- US 20170196547 A
- US 4235244 A
- US 4329995 A
- WO 2013084945 A
- US 2021244623 A
- US 5788680 A

### Non-patent literature cited in the description

- **CHAWES BL ; EDWARDS MJ ; SHAMJI B ; WALKER C ; NICHOLSON GC ; TAN AJ ; FOLSGAARD NV ; BONNELYKKE K ; BISGAARD H ; HANSEL TT**. A novel method for assessing unchallenged levels of mediators in nasal epithelial lining fluid. *J Allergy Clin Immunol*, 2010, vol. 125, 1387-1389
- **FOLSGAARD NV ; CHAWES BL ; RASMUSSEN MA ; BISCHOFF AL ; CARSON CG ; STOKHOLM J ; PEDERSEN L ; HANSEL TT ; BONNELYKKE K ; BRIX S**. Neonatal cytokine profile in the airway mucosal lining fluid is skewed by maternal atopy. *Am J Respir Crit Care Med*, 2012, vol. 185, 275-280
- **WOLSK HM ; FOLSGAARD NV ; BIRCH S ; BRIX S ; HANSEL TT ; JOHNSTON SL ; KEBADZE T ; CHAWES BL ; BONNELYKKE K ; BISGAARD H**. Picornavirus-Induced Airway Mucosa Immune Profile in Asymptomatic Neonates. *J Infect Dis*, 2016, vol. 213, 1262-1270
- **MELO JT, JR. ; TUNSTALL T ; PIZZICHINI MMM ; MAURICI R ; ROCHA CC ; DAL-PIZZOL F**. Goncalves J, Hansel TT, Thwaites RS, Pizzichini E. IL-5 Levels in Nasosorption and Sputosorption Correlate with Sputum Eosinophilia in Allergic Asthma. *Am J Respir Crit Care Med*, 2019, vol. 199, 240-243
- **SCADDING GW ; CALDERON MA ; BELLIDO V ; KOED GK ; NIELSEN NC ; LUND K ; TOGIAS A ; PHIPPARD D ; TURKA LA ; HANSEL TT**. Optimisation of grass pollen nasal allergen challenge for assessment of clinical and immunological outcomes. *J Immunol Methods*, 2012, vol. 384, 25-32
- **LEAKER BR ; MALKOV VA ; MOGG R ; RUDDY MK ; NICHOLSON GC ; TAN AJ ; TRIBOULEY C ; CHEN G ; DE LEPELEIRE I ; CALDER NA**. The nasal mucosal late allergic reaction to grass pollen involves type 2 inflammation (IL-5 and IL-13), the inflammasome (IL-1beta), and complement. *Mucosal Immunol*, 2017, vol. 10, 408-420
- **THWAITES RS ; GUNAWARDANA NC ; BROICH V ; MANN EH ; AHNSTROM J ; CAMPBELL GA ; LINDSLEY S ; SINGH N ; TUNSTALL T ; LANE DA**. Biphasic activation of complement and fibrinolysis during the human nasal allergic response. *J Allergy Clin Immunol*, 2018, vol. 141, 1892-1895
- **NICHOLSON GC ; KARIYAWASAM HH ; TAN AJ ; HOHLFELD JM ; QUINN D ; WALKER C ; RODMAN D ; WESTWICK J ; JURCEVIC S ; KON OM**. The effects of an anti-IL-13 mAb on cytokine levels and nasal symptoms following nasal allergen challenge. *J Allergy Clin Immunol*, 2011, vol. 128, 800-807
- **DHARIWAL J ; KITSON J ; JONES RE ; NICHOLSON G ; TUNSTALL T ; WALTON RP ; FRANCOMBE G ; GILBERT J ; TAN AJ ; MURDOCH R**. Nasal Lipopolysaccharide Challenge and Cytokine Measurement Reflects Innate Mucosal Immune Responsiveness. *PLoS One*, 2015, vol. 10, e0135363
- **HANSEL TT ; TUNSTALL T ; TRUJILLO-TORRALBO MB ; SHAMJI B ; DEL-ROSARIO A ; DHARIWAL J ; KIRK PDW ; STUMPF MPH ; KOOPMANN J ; TELCIAN A**. A Comprehensive Evaluation of Nasal and Bronchial Cytokines and Chemokines Following Experimental Rhinovirus Infection in Allergic Asthma: Increased Interferons (IFN-gamma and IFN-lambda) and Type 2 Inflammation (IL-5 and IL-13). *EBioMedicine*, 2017, vol. 19, 128-138
- **THWAITES RS ; ITO K ; CHINGONO JMS ; COATES M ; JARVIS HC ; TUNSTALL T ; ANDERSON-DRING L ; CASS L ; RAPEPORT G ; OPENSHAW PJ**. Nasosorption as a Minimally Invasive Sampling Procedure: Mucosal Viral Load and Inflammation in Primary RSV Bronchiolitis. *J Infect Dis*, 2017, vol. 215, 1240-1244

- **THWAITES RS** ; **COATES M** ; **ITO K** ; **GHAZALY M** ; **FEATHER C** ; **ABDULLA F** ; **TUNSTALL T** ; **JAIN P** ; **CASS L** ; **RAPEPORT G**. Reduced Nasal Viral Load and IFN Responses in Infants with Respiratory Syncytial Virus Bronchiolitis and Respiratory Failure. *Am J Respir Crit Care Med*, 2018, vol. 198, 1074-1084
- **HABIBI MS** ; **THWAITES RS** ; **CHANG M** ; **JOZWIK A** ; **PARAS A** ; **KIRSEBOM F** ; **VARESE A** ; **OWEN A** ; **CUTHBERTSON L** ; **JAMES P**. Neutrophilic inflammation in the respiratory mucosa predisposes to RSV infection. *Science*, 2020, 370
- **JHA A** ; **DUNNING J** ; **TUNSTALL T** ; **THWAITES RS** ; **HOANG LT** ; **INVESTIGATORS M** ; **KON OM** ; **ZAMBON MC** ; **HANSEL TT** ; **OPENSHAW PJ**. Patterns of systemic and local inflammation in patients with asthma hospitalised with influenza. *Eur Respir J*, 2019, 54
- **PROGATZKY F** ; **JHA A** ; **WANE M** ; **THWAITES RS** ; **MAKRIS S** ; **SHATTOCK RJ** ; **JOHANSSON C** ; **OPENSHAW PJ** ; **BUGEON L** ; **HANSEL TT**. Induction of innate cytokine responses by respiratory mucosal challenge with R848 in zebrafish, mice, and humans. *J Allergy Clin Immunol*, 2019, vol. 144, 342-345
- **JHA A** ; **THWAITES RS** ; **TUNSTALL T** ; **KON OM** ; **SHATTOCK RJ** ; **HANSEL TT** ; **OPENSHAW PJM**. Increased nasal mucosal interferon and CCL13 response to a TLR7/8 agonist in asthma and allergic rhinitis. *J Allergy Clin Immunol*, 2021, vol. 147, 694-703
- **THWAITES RS** ; **SANCHEZ SEVILLA URUCHURTU A** ; **SIGGINS MK** ; **LIEW F** ; **RUSSELL CD** ; **MOORE SC** ; **FAIRFIELD C** ; **CARTER E** ; **ABRAMS S** ; **SHORT CE**. Inflammatory profiles across the spectrum of disease reveal a distinct role for GM-CSF in severe COVID-19. *Sci Immunol*, 2021, vol. 6